# EUROPEAN PATENT SPECIFICATION

(11) **EP 2 003 453 B1**
(45) Date of publication and mention of the grant of the patent: **26.12.2012**
(21) Application number: 07736980.9
(22) Date of filing: 29.03.2007
(51) Int. Cl.: G01N 33/573, C12Q 1/32, C12Q 1/48, G01N 33/577

(54) **METHOD FOR DETECTION, DETERMINATION OR PREDICTION OF HEPATIC DISORDER**
VERFAHREN ZUM NACHWEIS, ZUR BESTIMMUNG ODER VORHERSAGE EINER LEBERERKRANKUNG
MÉTHODE POUR DÉTECTER, DÉTERMINER OU PRÉDIRE UN TROUBLE HÉPATIQUE

(30) Priority: 29.03.2006 JP 2006092169
(43) Date of publication of application: 17.12.2008
(73) Proprietor: YAMASA CORPORATION, Choshi-shi, Chiba 288-0056 (JP)
(72) Inventor: MURAYAMA, Hiroshi, Choshi-shi, Chiba 288-0056 (JP)
(74) Representative: MERH-IP Matias Erny Reichl Hoffmann
(86) International application number: PCT/JP2007/000323
(87) International publication number: WO 2007/122799

(56) References cited:
- EP-A- 1 619 257
- TAKASE S ET AL: "Biochemical markers of chronic alcoholism" ALCOHOL, PERGAMON PRESS, LONDON, GB, vol. 2, no. 3, 1 May 1985 (1985-05-01), pages 405-410, XP024400046 ISSN: 0741-8329 [retrieved on 1985-05-01]
- WATANABE YOSHIKO ET AL: "CLINICAL EVALUATION OF SERUM ORNITHINE CARBAMOYLTRANSFERASE BY ENZYME-LINKED IMMUNOSORBENT ASSAY IN PATIENTS WITH LIVER DISEASES" ENZYME PROTEIN, KARGER, BASEL, CH, vol. 48, no. 1, 1 January 1994 (1994-01-01), pages 18-26, XP008075586 ISSN: 1019-6773
- VAN WAES L ET AL: "Glutamate dehydrogenase: a reliable marker of liver cell necrosis in the alcoholic." BRITISH MEDICAL JOURNAL 10 DEC 1977, vol. 2, no. 6101, 10 December 1977 (1977-12-10), pages 1508-1510, XP002518718 ISSN: 0007-1447
- IMAI T.: 'Glutaminic Acid Dassuiso Koso (GLDH)' JAPANESE JOURNAL OF CLINICAL MEDICINE vol. 62, no. 11, 2004, pages 426 - 429, XP003012973
- MAEKAWA M.: 'Ornithine Carbamoyl Transferase (OCT)' JAPANESE JOURNAL OF CLINICAL MEDICINE vol. 62, no. 11, 2004, pages 459 - 462, XP003012974
- MURAYAMA HIROSHI ET AL: "Marked elevation of serum mitochondrion-derived markers in mild models of non-alcoholic steatohepatitis in rats.", JOURNAL OF GASTROENTEROLOGY AND HEPATOLOGY FEB 2009 LNKD- PUBMED:18823438, vol. 24, no. 2, February 2009 (2009-02), pages 270-277, ISSN: 1440-1746
- MURAYAMA HIROSHI ET AL: "Serum ornithine carbamyltransferase reflects hepatic damage in diabetic obese mice.", JOURNAL OF GASTROENTEROLOGY AND HEPATOLOGY FEB 2010 LNKD- PUBMED:19793175, vol. 25, no. 2, February 2010 (2010-02), pages 413-419, ISSN: 1440-1746
- TOKUSHIGE KATSUTOSHI ET AL: "Clinical significance of serum ornithine carbamoyltransferase in patients with non-alcoholic steatohepatitis.", HEPATOLOGY RESEARCH : THE OFFICIAL JOURNAL OF THE JAPAN SOCIETY OF HEPATOLOGY SEP 2009 LNKD- PUBMED:19712272, vol. 39, no. 9, September 2009 (2009-09), pages 939-943, ISSN: 1386-6346

## Description

### Technical Field

The present invention relates to a method for detecting a hepatic disorder of a subject, in which the amount of a mitochondrion-derived protein contained in a blood sample from the subject is measured, and a hepatic disorder of the subject is detected on the basis of the measurement thereof. The present invention relates to a method for detecting a hepatic disorder derived from metabolic syndrome and/or non-alcoholic fatty liver disease; in particular, for example, non-alcoholic steatohepatitis, hepatic fibrosis, cirrhosis, or liver cancer.
The present disclosure also relates to, for example, a method for predicting progress from metabolic syndrome or non-alcoholic fatty liver disease to non-alcoholic steatohepatitis, hepatic fibrosis, cirrhosis, or liver cancer.

### Background Art

Fatty liver diseases which are not caused by excessive alcohol intake are called "non-alcoholic fatty liver disease" (hereinafter may be abbreviated as "NAFLD"), which encompass metabolic-syndrome-induced fatty liver disease described hereinbelow. Among NAFLD, a pathological condition further involving inflammation of the liver is called "non-alcoholic steatohepatitis" (hereinafter may be abbreviated as "NASH"). NASH is a chronic liver disease which may gradually progress to hepatic fibrosis, cirrhosis, or liver cancer by any factor such as oxidative stress.

Metabolic syndrome, also referred to as abnormal metabolic regulation, which is a combination of visceral fat accumulation with two or more of hyperglycemia, hypertension, and hyperlipidemia, is generally considered a risk factor for inducing arteriosclerotic diseases or fatty liver diseases, and thus is required to be prevented or treated.

NASH is considered the hepatic manifestation of metabolic syndrome. Patients having metabolic syndrome or diabetes are considered to have a high risk to develop NASH in combination with such a pathological condition.

Although NASH is described above as a pathological condition further involving inflammation of the liver among NAFLD, NASH is not clearly defined at present. For example, in some cases, NASH may progress to hepatic fibrosis, cirrhosis, or liver cancer, although inflammatory cells do not infiltrate the liver. Some researchers have considered that hypertrophy of the hepatocyte (which is called "ballooning"), rather than inflammation of the liver, plays an important role in progress of NASH to hepatic fibrosis, cirrhosis, or liver cancer. In addition, it has been reported that progress to cirrhosis is not observed in most of NASH patients. Therefore, as used herein, "NASH" collectively refers to the state where the liver develops a hepatic disorder (e.g., inflammation, ballooning, fibrosis, cirrhosis, or cancer), or the state where the liver may induce such a pathological condition, and "NASH" is distinguished from "simple steatosis"; i.e., a condition in which fat is simply accumulated in the liver, and which does not progress to another hepatic-disorder-developing condition.

As indicated by its name, NASH is not caused by alcohol intake. However, NASH exhibits symptoms similar to those of alcoholic hepatitis. In advanced countries, NASH is considered the most frequent liver disease and one of severe diseases. Therefore, patients with NASH are required to undergo vigorous treatment, and accurate discrimination between simple steatosis and NASH is very important in the diagnosis thereof.

Conventionally, NASH has been diagnosed through diagnostic imaging (e.g., abdominal echography or computed tomography), or through a blood test for examining change in amount of a marker for diagnosis of hepatic disorders (hereinafter the marker may be referred to as a "hepatic injury marker") (e.g., glutamic-oxaloacetic transaminase (GOT) or glutamic-pyruvic transaminase (GPT)). However, the level of the aforementioned hepatic injury marker does not necessarily fall within an abnormal range in all NASH patients, and thus the definite diagnosis of NASH must be established by liver biopsy.

However, liver biopsy is not suitable as a general examination technique, since liver biopsy is highly invasive to patients and requires an intricate process, and the pathological results must be determined by skilled experts. Therefore, demand has arisen for development of a highly reliable examination method for diagnosis of NASH in place of the aforementioned examination methods.

Hitherto, there have been reported many studies on change in hepatic injury marker blood levels by use of animal models. Most of these studies are experiments employing rats or mice fed with a choline-deficient diet or a methionine-and choline-deficient diet, or rats or mice to which a drug inducing symptoms similar to those of NASH has been administered. These experiments generally employ GPT as a hepatic injury marker. In these animal models, necrosis occurs in the liver, and an increase in GPT level is observed. However, it has been known that, in human NASH patients, NASH may progress to cirrhosis without an increase in GPT level. Therefore, GPT is not necessarily suitable as a hepatic injury marker for diagnosis of NASH.

Also, there have been reported rat experiments in which, for example, ornithine carbamoyltransferase (OCT) is employed as a hepatic injury marker other than GPT, and the concentration of OCT is measured (see, for example, Non-Patent Documents 1 and 2).

Non-Patent Document 1: Biosci. Biotechnol. Biochem. 2001; 65 (4): 935-938
Non-Patent Document 2: Toxicol. Lett. 2000; 114 (1-3): 163-171

Since OCT is localized in mitochondria, leakage of OCT into blood requires passing through the cell membrane and also the two mitochondrial membranes, and thus OCT leaks into blood slower than, for example, GPT, which is derived from cytoplasm. Therefore, OCT has not been considered a more effective marker than GPT, and no data have been reported on such applicability of OCT. As has been reported so far, OCT is used merely for the purpose of supporting occurrence of a disorder in the liver by utilizing high liver-specificity of OCT, or for the purpose of determining whether or not the disorder is so severe that it affects mitochondria. Thus, it has not hitherto been described that the mitochondrion-derived protein contained in blood is a highly detectable and excellent marker as compared with GPT. That is, GPT has been recognized as a marker for detecting hepatic disorders such as NASH at high sentitivity. Therefore, almost all of the related papers, etc. which have been reported hitherto disclose use of GPT as a hepatic injury marker.

Patent Document 1 discloses a method for diagnosing the presence or severity of hepatic fibrosis on the basis of the presence or level of α-2-macroglobulin, hyaluronic acid, or metalloproteinase-1 inhibitor, which serves as a hepatic injury marker.
Patent Document 2 discloses a method for the differential diagnosis of NASH by determining phospholipid contained in peripheral blood and the fatty acid composition of the phospholipid.
Patent Document 3 discloses a method for diagnosing NASH by quantifying, in a hepatic tissue sample, the level of a protein selected from among apolipoprotein A1, mitochondrial ATPase β subunit, leukotriene A4 hydrolase, keratin 18, guanidinoacetate N-methyltransferase, superoxide dismutase, albumin, antioxidant protein 2 (isoform 1), prohibitin, methionine adenosyltransferase, long-chain acyl-CoA dehydrogenase, selenium-binding protein, antioxidant protein 2 (isoform 2), and combinations thereof.
Patent Document 4 discloses a method for diagnosing NASH by determining the type III procollagen N-terminal peptide evel.
EP1619257 discloses deregulated GDH levels in NASH.

Patent Document 1: Japanese Kohyo Patent Publication No. 2005-519271
Patent Document 2: WO 2005/109006
Patent Document 3: WO 2004/55520
Patent Document 4: Japanese Patent Application Laid-Open (*kokai*) No. 2006-29919

### Disclosure of the Invention

As described above, a blood test employing, as an index, the level of GOT or GPT, which is a hepatic injury marker, is not satisfactory in that NASH may fail to be detected when the index represented by such a hepatic injury marker indicates negative or weakly positive.

There has also been reported a test method for the diagnosis of NASH employing a hepatic injury marker other than GOT or GPT. The test method requires an intricate measuring process and, in some cases, requires measurement of a plurality of markers. Thus, the test method is not necessarily convenient means.

When the level of such a hepatic injury marker in a patient falls within an abnormal range, liver biopsy may be carried out. However, liver biopsy is highly invasive to patients and requires an intricate process, and the pathological results must be determined by skilled experts. Therefore, liver biopsy is not suitable as a general examination technique, and even when the patient is suspected of having NASH, liver biopsy is not carried out in some cases.

In view of the foregoing, an object of the present invention is to provide a method in which the mitochondrion-derived protein level of a blood sample from a subject is measured, and whether or not the subject has a hepatic disorder (in particular, NASH) is determined on the basis of the measured mitochondrion-derived protein level.

In order to achieve the aforementioned object, the present inventor has conducted extensive studies, and as a result has found that, for example,
(A) in a rodent animal model fed with a choline-deficient diet or a methionine- and choline-deficient diet, and developing symptoms similar to those of NASH, GPT level does not significantly increase as compared with a control group;
(B) in many human NASH patients, GPT level is observed to fall within a normal range;
(C) in an NASH case; in particular, a mild NASH case with no or slight increase in GPT level, there is a significant increase in blood level of a mitochondrion-derived protein such as ornithine carbamoyltransferase (OCT) or glutamate dehydrogenase (GDH); and
(D) measurement of a mitochondrion-derived protein is useful for detecting not only NASH but also metabolic syndrome (including diabetes). The present inventor has first found that the amount of leakage of a mitochondrion-derived protein into blood increases in an NASH case with no or only a slight increase in GPT level. In connection therewith, the present inventor has found that NASH, which is a hepatic disorder, can be examined by determining the mitochondrion-derived protein blood level. The present invention has been accomplished on the basis of this finding. The scope of protection is defined by the claims. Accordingly, the present disclosure provides the following.

(1) A method for detecting a hepatic disorder, comprising measuring the level of mitochondrion-derived protein in a blood sample from a subject, and detecting a hepatic disorder of the subject on the basis of the measured mitochondrion-derived protein level.
(2) A detection method as described in (1), wherein the hepatic disorder of the subject is a hepatic disorder occurring via a mechanism in which glutamic-pyruvic transaminase level is less likely to increase, or a hepatic disorder at such a degree that glutamic-pyruvic transaminase level does not increase.
(3) A detection method as described in (1), wherein the hepatic disorder of the subject is derived from metabolic syndrome and/or non-alcoholic fatty liver disease.
(4) A detection method as described in (1), wherein the hepatic disorder of the subject is non-alcoholic steatohepatitis, hepatic fibrosis, cirrhosis, or liver cancer.
(5) A method for determining a hepatic disorder, comprising measuring the level of mitochondrion-derived protein level in a blood sample from a subject; comparing the measured mitochondrion-derived protein level with an averaged value of mitochondrion-derived protein levels of blood samples from healthy volunteers; and, when the mitochondrion-derived protein level of the subject is higher than the averaged value of mitochondrion-derived protein levels of the healthy volunteers, determining that the subject has a hepatic disorder derived from metabolic syndrome and/or non-alcoholic fatty liver disease.
(6) A method for predicting a hepatic disorder of a patient with metabolic syndrome and/or non-alcoholic fatty liver disease, the method comprising employing a mitochondrion-derived protein as a hepatic injury marker.
(7) A method for predicting progress of a hepatic disorder, comprising tracing change over time in level of mitochondrion-derived protein in a blood sample from a patient with metabolic syndrome and/or non-alcoholic fatty liver disease.
(8) A method for determining a therapeutic effect on a hepatic disorder, comprising measuring the level of mitochondrion-derived protein level in a blood sample from a patient with metabolic syndrome and/or non-alcoholic fatty liver disease, and determining a therapeutic effect on a hepatic disorder on the basis of the measured mitochondrion-derived protein level.
(9) A method as described in any of (1) to (8), wherein the mitochondrion-derived protein is a protein which exists specifically in the liver and/or a protein which exists abundantly in the liver.
(10) A method as described in any of (1) to (8), wherein the mitochondrion-derived protein is ornithine carbamoyltransferase and/or glutamate dehydrogenase.
(11) A method as described in any of (1) to (8), wherein the mitochondrion-derived protein level is determined by measuring the concentration or activity of the protein.
(12) A method as described in any of (1) to (8), wherein the mitochondrion-derived protein level is measured through an immunological technique or an enzymatic technique.

A hepatic disorder derived from NAFLD can be detected, and NASH can be accurately discriminated from simple steatosis. Also, the present disclosure is useful as a definite diagnosis of NASH or as an auxiliary examination for determining whether a definite diagnosis (e.g., liver biopsy) is needed or not.

Furthermore, progress of NASH to hepatic inflammation, hepatic fibrosis, cirrhosis, liver cancer, or the like can be predicted by tracing change over time in mitochondrion-derived protein level of a blood sample from an NAFLD patient. Also, the present disclosure can be applied to determination of a therapeutic effect on a hepatic disorder.

In addition, the present methods can detect not only a hepatic disorder derived from NAFLD, but also a hepatic disorder derived from metabolic syndrome. Therefore, the present methods can also be applied to detection of metabolic syndrome itself, or prediction of progress of a metabolic-syndrome-derived hepatic disorder such as NASH (i.e., hepatic inflammation, hepatic fibrosis, cirrhosis, or liver cancer).

### Brief Description of the Drawings

[Fig. 1] Fig. 1 is a graph showing the relationship between TBARS concentration and hours after administration of tetracycline.
[Fig. 2] Fig. 2 is a graph showing the relationship between hepatic injury marker concentration and hours after administration of tetracycline.
[Fig. 3] Fig. 3 is a graph showing change over time (age in week) in relative concentration of serum OCT and GPT of male ob/ob mice.

### Best Modes for Carrying Out the Invention

No particular limitation is imposed on the test sample for measurement, so long as the test sample is a blood-derived sample such as blood, serum, or plasma.

A characteristic feature of the method of the present disclosure resides in that the mitochondrion-derived protein level is measured for detection of a hepatic disorder such as NASH. Examples of the mitochondrion-derived protein include OCT and/or GDH.

The OCT or GDH level of a blood sample may be determined on the basis of the concentration or activity of the protein, which may be measured through a known method. For example, when the level of OCT or GDH is measured through an immunological technique such as ELISA, there may be employed, for example, the highly sensitive OCT assay method developed by the present inventor (Patent Document 5: WO 2006/73073) or a known method (Enzyme Protein (1994-95) 48: 10-17).

When the level of OCT or GDH is measured through an enzymatic technique, there may be employed, for example, a commercially available kit (e.g., OCT-Test Wako, product of Wako Pure Chemical Industries, Ltd.) or a known method (Clin. Chim. Acta (1976) 67: 145-152, Anal. Lett. (1993) 26: 475-486, or Eur. J. Clin. Chem. Clin. Biochem. (1992) 30: 493-502).

The mitochondrion-derived protein level of a blood sample from a subject is measured through any of the aforementioned methods or by use of the aforementioned commercially available kit, and the thus-measured level is compared with an averaged value of mitochondrion-derived protein levels of blood samples from healthy volunteers. When the mitochondrion-derived protein level of the subject is higher than the average of the an averaged value of mitochondrion-derived protein levels of the healthy volunteers, the subject is determined to be suspected of having a hepatic disorder; specifically, a hepatic disorder (e.g., NASH) derived from metabolic syndrome and/or NAFLD. In contrast, when the mitochondrion-derived protein level of the subject is equal to or lower than the averaged value of mitochondrion-derived protein levels of the healthy volunteers, the subject is determined not to have a hepatic disorder (e.g., NASH).

When the OCT or GDH concentration is determined through an immunological technique such as ELISA, an antibody to OCT or GDH is employed. The antibody may be prepared through a customary method (see, for example, Patent Document 5), and may be a monoclonal or polyclonal antibody. Alternatively, the antibody may be an antibody fragment having a binding ability to OCT or GDH (e.g., Fab or F(ab')₂). Examples

The present invention will next be described by way of examples.

### Example 1: Measurement of hepatic injury marker blood levels of SD rats fed with choline-deficient diet

Sixteen male SD rats (8-week old) were divided into two groups. SD rats of one group were fed with a choline-deficient (CD) diet, and SD rats of the other group were fed with a choline-supplemented (CS) diet. Four weeks later, blood was collected from each SD rat under anesthesia. In addition, a portion of the liver of each SD rat was collected, and fixed in formalin, followed by hematoxylin-eosin staining. Thus, the degree of a hepatic disorder was histopathologically examined. SD rats fed with the CD diet for eight weeks were also examined in a manner similar to that described above.

Tables 1 and 2 show the results of measurement of the serum level and activity of mitochondrion-derived proteins (OCT and GDH) and cytoplasm-derived proteins (GPT, GOT, guanase (GU), isocyanate dehydrogenase (ICD), sorbitol dehydrogenase (SDH), and lactate dehydrogenase (LDH)). The levels of GPT, GOT, GU, ICD, SDH, LDH, and GDH were measured through an enzymatic technique, and the OCT level was measured through ELISA.

As shown in Table 1, in the CD diet group, the levels of OCT and GDH, which are mitochondrion-derived proteins, significantly increased. In contrast, as shown in Table 2, there was no significant difference in level of each cytoplasm-derived protein between the CD diet group and the CS diet group. These data indicate that NASH is effectively detected on the basis of the blood mitochondrion-derived protein level.

**[Table 1]**

| | OCT (ng/mL) | GDH (IU/L) |
|---|---|---|
| CS diet group | 23.4 ± 9.1 | 19.8 ± 2.5 |
| CD diet group | 115.6 ± 29.0* | 78.7 ± 17.7* |

| | | |
|---|---|---|
| *: p < 0.05 (Student t-test) | | |

**[Table 2]**

| | GPT (Karmen unit) | GOT (Karmen unit) | GU (IU/L) | ICD (IU/L) | SDH (IU/L) | LDH (Wroblewski unit) |
|---|---|---|---|---|---|---|
| CS diet group | 14.3 ± 1.3 | 86.8 ± 5.7 | 80.6 ± 3.3 | 3.0 ± 0.6 | 4.7 ± 1.0 | 2109 ± 179 |
| CD diet group | 29.1 ± 7.0 | 109.8 ± 13.4 | 75.2 ± 5.1 | 3.1 ± 1.0 | 8.0 ± 1.4 | 1951 ± 301 |

| | | | | | | |
|---|---|---|---|---|---|---|
| No significant difference in each protein (Student t-test) | | | | | | |

Through the histopathological examination, four weeks after initiation of feeding of the CD diet, accumulation of fat was observed in the liver of the rats of the CD diet group, but no marked hepatic disorder was observed. However, eight weeks after initiation of feeding of the CD diet, hepatic fibrosis was observed in the rats of the CD diet groups. These data indicate that the liver of SD rats is damaged by feeding of the CD diet, and a mitochondrion-derived protein (OCT or GDH) is useful as a hepatic injury marker.

### Example 2: Measurement of blood hepatic injury marker levels of tetracycline-administered rats

Mitochondrial disorder or oxidative stress associated therewith is thought to be involved in the mechanism of development of NASH. As has been known, even in human, a drug which induces mitochondrial disorder may cause a lesion similar to NASH. This lesion is also called "drug-induced NASH." In Example 2, there was performed measurement over time of the blood hepatic injury marker levels of rats with an acute hepatic disorder caused by administration of tetracycline, as well as the amount of thiobarbituric acid reactive substance (TBARS) (which is considered an index of oxidative stress) contained in liver tissue.

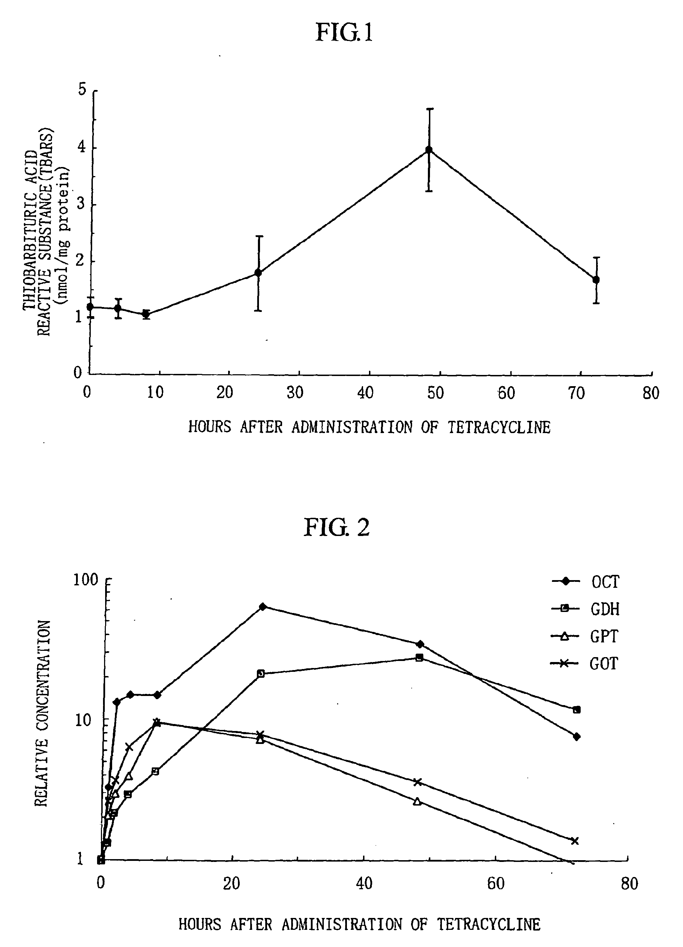

Male Wistar rats (8-week old) were employed, and tetracycline hydrochloride (192 mg/kg) was intraperitoneally administered to each rat. Subsequently, blood was collected from each rat under anesthesia, followed by removal of the liver. 20 mM Phosphate buffer (4 mL) was added to liver tissue (1 g), followed by homogenization, to thereby prepare a liver tissue sample. The thus-prepared sample was subjected to measurement of TBARS. Fig. 1 shows change in TBARS concentration of liver tissue, and Fig. 2 shows change in blood level of hepatic injury markers after administration of tetracycline. The value at each measurement point in time corresponds to the average of data obtained from six Wistar rats (8-week old).

As shown in Fig. 1, the TBARS concentration of liver tissue started to increase 24 hours after administration of tetracycline, and reached a peak 48 hours after administration of tetracycline. As shown in Fig. 2, the blood GPT and GOT levels, which are cytoplasm-derived proteins, reached a peak eight hours after administration of tetracycline, and then gradually decreased. In contrast, the blood level of OCT, which is a mitochondrion-derived protein, reached a peak four hours after administration of tetracycline, and reached another peak 24 hours after administration of tetracycline. The blood GDH level reached a peak 48 hours after administration of tetracycline. The second peak of the OCT level or the peak of the GDH level corresponds to the peak of the TBARS concentration, which elevates in accordance with an increase in oxidative stress in the liver. Thus, an increase in blood OCT or GDH level is thought to reflect oxidative stress associated with mitochondrial disorder. In contrast, the peak of the GPT or GOT level does not correspond to the peak of the TBARS concentration.

### Example 3: Measurement of blood hepatic injury marker levels of KK-Ay mice fed with high-fat diet

A KK-Ay mouse is an obese and diabetic mouse established by transduction of the obese gene Ay into KK mouse, which has a genetic background that makes it more likely to develop diabetes. As has been reported, when KK-Ay mice are fed with a high-fat diet, the adiponectin (i.e., good adipokine) level decreases, and the TNF-α (i.e., bad adipokine) level increases. Therefore, a KK-Ay mouse fed with a high-fat diet is considered an animal model of metabolic syndrome.
In Example 3, the blood level of a mitochondrion-derived protein was measured in KK-Ay mice fed with a high-fat diet.

Ten male KK-Ay mice were divided into two groups (five mice each). Mice of the two groups were respectively fed with a control diet and a high-fat diet for three weeks, followed by measurement of blood OCT and GPT levels. The results are shown in Table 3.

As shown in Table 3, in the high-fat diet group, only OCT level significantly increased. These data indicate that OCT is more preferred than GPT as an index for detecting NASH, which is a hepatic disorder associated with metabolic syndrome. An increase in OCT level in KK-Ay mice fed with the high-fat diet (i.e., model of metabolic syndrome) supports the relationship between metabolic syndrome and NASH. Thus, it is shown that the blood mitochondrion-derived protein level is effectively employed not only for diagnosis of NASH, but also for diagnosis of metabolic syndrome.

**[Table 3]**

| | OCT (ng/mL) | GPT (Karmen unit) |
|---|---|---|
| Control diet group | 15.9 ± 1.1 | 22.1 ± 2.5 |
| High-fat diet group | 20.6 ± 2.1* | 25.4 ± 0.6 |

| | | |
|---|---|---|
| *: p < 0.05 (Student t one-tailed test) | | |

These data indicate that the method employing a mitochondrion-derived protein (e.g., OCT or GDH) as a hepatic injury marker can more sensitively detect mitochondrial disorder or oxidative stress associated therewith (which is considered to be involved in the mechanism of development of NASH), as compared with the case where a cytoplasm-derived protein (e.g., GPT or GOT) is employed; i.e., the method is effective for diagnosis of NASH.

### Example 4: Measurement of blood hepatic injury marker levels of ob/ob mice

NASH is considered to be the hepatic manifestation of metabolic syndrome, and 80 to 90% of NASH patients have visceral obesity. Most of diabetic patients have hepatic steatosis, and are considered to have a high risk to develop NASH, as compared with healthy people. Ob/ob mouse, which is a mouse model of type 2 diabetes, has extreme obesity and hepatic steatosis, and exhibits sensitivity to hepatotoxicity such as lipopolysaccharide or alcohol intake. Therefore, ob/ob mice were employed as a model of NASH. In Example 4, the blood mitochondrion-derived protein level of ob/ob mice was evaluated.

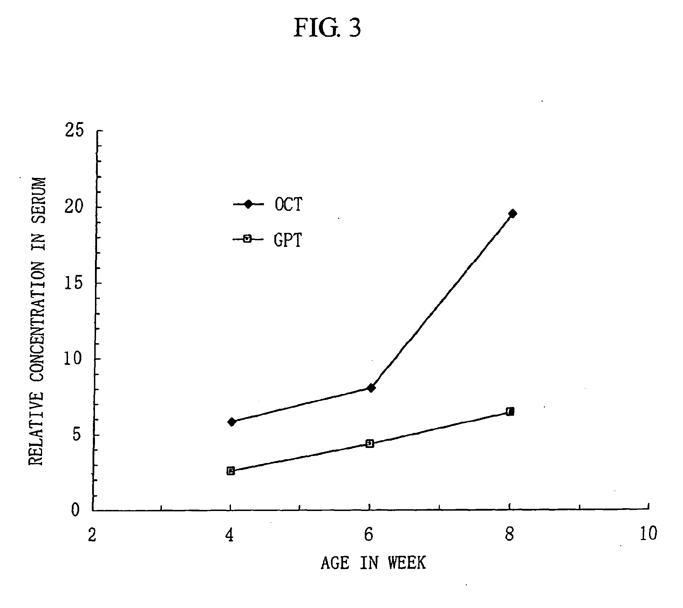

Five male ob/ob mice and five male control mice were employed, and blood OCT and GPT levels were measured from 4-week old to 8-week old. The results are shown in Fig. 3. As shown in Fig. 3, at 4-week old, at which obesity became noticeable, the rate of increase in blood OCT level in the ob/ob mice was about six times that in the control mice, and the rate of increase in blood OCT level was twice or more the rate of increase in blood GPT level. At 6- and 8-week old, the rate of increase in blood OCT level was higher as obesity became more noticeable.

As has been known, in ob/ob mice (i.e., diabetic mouse model with high obesity), an increase in blood glucose level, which is an index of diabetes, is less likely to be observed. An increase in blood hepatic injury marker level of the ob/ob mice indicates that metabolic syndrome with obesity or hepatic steatosis may progress to a hepatic disorder, even in the case of mild diabetes. Thus, it is shown that measurement of the blood mitochondrion-derived protein level of the ob/ob mice is effective for detecting not only NASH but also metabolic syndrome (including diabetes).

In the ob/ob mice having visceral obesity, the rate of increase in blood level of OCT (i.e., a mitochondrion-derived protein) over time is higher, as compared with the case of GPT, which is generally widely used as a hepatic injury marker. This suggests that OCT is more effective than GPT for diagnosis of NASH.

The present disclosure has first realized use of a mitochondrion-derived protein as a hepatic injury marker for predicting a hepatic disorder of a patient with metabolic syndrome and/or non-alcoholic fatty liver disease.

According to the present disclosure, progress of a hepatic disorder can be predicted by tracing change over time in mitochondrion-derived protein level of a blood sample from a patient with metabolic syndrome and/or non-alcoholic fatty liver disease.

In addition, according to the present disclosure, a therapeutic effect on a hepatic disorder can be determined on the basis of the mitochondrion-derived protein level of a blood sample from a patient with metabolic syndrome and/or non-alcoholic fatty liver disease.

## Claims

1. A method for detecting a hepatic disorder occurring via a mechanism in which glutamic-pyruvic transaminase level does not significantly increase, or a hepatic disorder at such a degree that glutamic-pyruvic transaminase level does not increase, said method comprising measuring the level of mitochondrion-derived protein level in a blood sample from a subject; comparing the measured mitochondrion-derived protein level with an averaged value of mitochondrion-derived protein levels of blood samples from healthy volunteers; and, when the mitochondrion-derived protein level of the subject is higher than the averaged value of mitochondrion-derived protein levels of the healthy volunteers, determining that the subject has a hepatic disorder derived from metabolic syndrome and/or non-alcoholic fatty liver disease, wherein the mitochondrion-derived protein is ornithine carbamoyltransferase and/or glutamate dehydrogenase.

2. A detection method as described in claim 1, wherein the hepatic disorder of the subject is non-alcoholic steatohepatitis, hepatic fibrosis, cirrhosis, or liver cancer.

3. The method of any preceding claim for predicting a hepatic disorder of a patient with metabolic syndrome and/or non-alcoholic fatty liver disease, the method comprising employing a mitochondrion-derived protein as a hepatic injury marker.

4. The method of any preceding claim for predicting progress of a hepatic disorder, comprising tracing change over time in level of mitochondrion-derived protein in a blood sample from a patient with metabolic syndrome and/or non-alcoholic fatty liver disease.

5. The method of any preceding claim for determining a therapeutic effect on a hepatic disorder on the basis of the measured mitochondrion-derived protein level.

6. A method as described in any of claims 1 to 5, wherein the mitochondrion-derived protein is a protein which exists specifically in the liver and/or a protein which exists abundantly in the liver.

7. A method as described in any of claims 1 to 6, wherein the mitochondrion-derived protein level is determined by measuring the concentration or activity of the protein.

8. A method as described in any of claims 1 to 7, wherein the mitochondrion-derived protein level is measured through an immunological technique or an enzymatic technique.

## Patentansprüche

1. Verfahren zum Nachweis einer Lebererkrankung, der über einen Mechanismus erfolgt, bei dem ein Glutamat-Pyruvat-Transaminase-Spiegel nicht bedeutend ansteigt, oder einer Lebererkrankung von einem solchen Grad, dass der Glutamat-Pyruvat-Transaminase-Spiegel nicht ansteigt, wobei das Verfahren umfasst: Messen des Spiegels eines mitochondrium-abgeleiteten Proteinspiegels in einer Blutprobe von einem Patienten; Vergleichen des gemessenen mitochondrium-abgeleiteten Proteinspiegels mit einem Mittelwert von mitochondrium-abgeleiteten Proteinspiegeln von Blutproben von gesunden Freiwilligen; und, wenn der mitochondrium-abgeleitete Proteinspiegel des Patienten höher als der Mittelwert von mitochondrium-abgeleiteten Proteinspiegeln der gesunden Freiwilligen ist, Bestimmen, dass der Patient eine Lebererkrankung hat, die von einem metabolischen Syndrom und/oder einer nicht-alkoholischen Fettleberkrankheit abgeleitet ist, wobei das mitochondrium-abgeleitete Protein Ornithin-Carbamoyltransferase und/oder Glutamatdehydrogenase ist.

2. Nachweisverfahren nach Anspruch 1, wobei die Lebererkrankung des Patienten nicht-alkoholische Steatohepatitis, Hepatitisfibrose, Cirrhose oder Leberkrebs ist.

3. Verfahren nach irgendeinem vorhergehenden Anspruch zur Vorhersage einer Lebererkrankung eines Patienten mit metabolischem Syndrom und/oder nicht-alkoholischer Fettleberkrankheit, wobei das Verfahren das Verwenden eines mitochondrium-abgeleiteten Proteins als Leberschädigungsmarkierung umfasst.

4. Verfahren nach irgendeinem vorhergehenden Anspruch zur Vorhersage des Fortschreitens einer Lebererkrankung, mit der Verfolgung einer Änderung des Spiegels von mitochondrium-abgeleitetem Protein im Lauf der Zeit in einer Blutprobe von einem Patienten mit metabolischem Syndrom und/oder nicht-alkoholischer Fettlebererkrankung.

5. Verfahren nach irgendeinem vorhergehenden Anspruch zum Bestimmen einer therapeutischen Wirkung auf eine Lebererkrankung auf der Grundlage des gemessenen mitochondrium-abgeleiteten Proteinspiegels.

6. Verfahren nach irgendeinem der Ansprüche 1 bis 5, wobei das mitochondrium-abgeleitete Protein ein Protein ist, das insbesondere in der Leber vorkommt und/oder ein Protein ist, das übermäßig in der Leber vorkommt.

7. Verfahren nach irgendeinem der Ansprüche 1 bis 6, wobei der mitochondrium-abgeleitete Proteinspiegel durch Messen der Konzentration oder Aktivität des Proteins bestimmt wird.

8. Verfahren nach irgendeinem der Ansprüche 1 bis 7, wobei der mitochondrium-abgeleitete Proteinspiegel durch eine immunologische Technik oder eine enzymatische Technik gemessen wird.

## Revendications

1. Procédé pour détecter un trouble hépatique qui se produit via un mécanisme dans lequel un niveau de transaminase glutamique-pyruvique n'augmente pas de manière significative, ou un trouble hépatique à un degré tel qu'un niveau de transaminase glutamique-pyruvique n'augmente pas, ledit procédé comprenant les opérations consistant à mesurer le niveau de protéine dérivée de mitochondrie dans un échantillon sanguin d'un sujet ; à comparer le niveau de protéine dérivée de mitochondrie avec une valeur moyenne de niveaux de protéine dérivée de mitochondrie dans des échantillons sanguins provenant de volontaires sains ; et, quand le niveau de protéine dérivée de mitochondrie du sujet est supérieur à la valeur moyenne des niveaux de protéine dérivée de mitochondrie des volontaires sains, à déterminer que le sujet a un trouble hépatique dérivé de ce syndrome métabolique et/ou d'une hépatite graisseuse non-alcoolique, dans lequel la protéine dérivée de mitochondrie est de l'ornithine carbamoyltransférase et/ou du glutamate déhydrogénase.

2. Procédé de détection selon la revendication 1, dans lequel le trouble hépatique du sujet est une stéatose hépatique non-alcoolique, une fibrose hépatique, une cirrhose ou un cancer du foie.

3. Procédé selon l'une quelconque des revendications précédentes pour prédire un trouble hépatique d'un patient avec un syndrome métabolique et/ou une hépatite graisseuse non-alcoolique, le procédé comprenant l'emploi d'une protéine dérivée de mitochondrie à titre de marqueur de lésion hépatique.

4. Procédé selon l'une quelconque des revendications précédentes pour prédire une progression d'un trouble hépatique, comprenant un suivi des changements au cours du temps du niveau de protéine dérivée de mitochondrie dans un échantillon sanguin provenant d'un patient avec un syndrome métabolique et/ou une hépatite graisseuse non-alcoolique.

5. Procédé selon l'une quelconque des revendications précédentes pour déterminer un effet thérapeutique sur un trouble hépatique en se basant sur le niveau mesuré de protéine dérivée de mitochondrie.

6. Procédé selon l'une quelconque des revendications 1 à 5, dans lequel la protéine dérivée de mitochondrie est une protéique qui existe spécifiquement dans le foie et/ou une protéine qui existe de façon abondante dans le foie.

7. Procédé selon l'une quelconque des revendications 1 à 6, dans lequel le niveau de protéine dérivée de mitochondrie est déterminé en mesurant la concentration ou l'activité de la protéine.

8. Procédé selon l'une quelconque des revendications 1 à 7, dans lequel le niveau de protéine dérivée de mitochondrie est mesuré via une technique immunologique ou une technique enzymatique.
